(19) **Europäisches Patentamt / European Patent Office / Office européen des brevets**

(11) **EP 4 406 575 B1**

(12) ## FASCICULE DE BREVET EUROPEEN

(45) Date de publication et mention de la délivrance du brevet:
**08.10.2025 Bulletin 2025/41**

(21) Numéro de dépôt: **23214552.4**

(22) Date de dépôt: **06.12.2023**

(51) Classification Internationale des Brevets (IPC):
**A61M 16/00** (2006.01)    **A61B 5/087** (2006.01)
**A61B 5/091** (2006.01)

(52) Classification Coopérative des Brevets (CPC):
**A61M 16/024; A61B 5/087; A61B 5/091;**
**A61B 5/4836; A61M 16/0069; A61M 16/125;**
**A61M 16/205;** A61M 16/204; A61M 2016/0027;
A61M 2016/0039; A61M 2202/0208;
A61M 2205/3331; A61M 2205/505; A61M 2205/583;
A61M 2205/8206;                    (Cont.)

(54) **VENTILATEUR MÉDICAL AVEC AFFICHAGE D'UNE INDICATION DE LA RECRUTABILITÉ DES POUMONS D'UN PATIENT**

MEDIZINISCHES BEATMUNGSGERÄT MIT ANZEIGE DER LUNGENREKRUTBARKEIT EINES PATIENTEN

MEDICAL VENTILATOR WITH DISPLAY OF PATIENT LUNG RECRUITABILITY INDICATION

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité: **24.01.2023 FR 2300650**

(43) Date de publication de la demande:
**31.07.2024 Bulletin 2024/31**

(73) Titulaire: **Air Liquide Medical Systems**
**92182 Antony Cedex (FR)**

(72) Inventeurs:
• **LESIMPLE, Arnaud**
  **92182 Antony Cedex (FR)**
• **DE BEAUFORT, Eloise**
  **92182 Antony Cedex (FR)**
• **RICHARD, Jean-Christophe**
  **92182 Antony Cedex (FR)**
• **BROC, Alexandre**
  **92182 Antony Cedex (FR)**

(74) Mandataire: **Air Liquide**
**L'Air Liquide S.A.**
**Direction de la Propriété Intellectuelle**
**75, Quai d'Orsay**
**75321 Paris Cedex 07 (FR)**

(56) Documents cités:
**US-A1- 2022 096 765**

• **CHEN LU ET AL: "Potential for Lung Recruitment Estimated by the Recruitment-to-Inflation Ratio in Acute Respiratory Distress Syndrome. A Clinical Trial", AMERICAN JOURNAL OF RESPIRATORY AND CRITICAL CARE MEDICINE, vol. 201, no. 2, 15 January 2020 (2020-01-15), US, pages 178 - 187, XP093066439, ISSN: 1073-449X, DOI: 10.1164/rccm.201902-0334OC**
• **HANS-JÖRG GILLMANN ET AL: "Association of radiological lung pattern and respiratory mechanics with potential for lung recruitment in patients with COVID-ARDS: a retrospective cohort study", EUROPEAN JOURNAL OF MEDICAL RESEARCH, BIOMED CENTRAL LTD, LONDON, UK, vol. 27, no. 1, 1 October 2022 (2022-10-01), pages 1 - 8, XP021308579, DOI: 10.1186/S40001-022-00821-W**
• **COUR MARTIN ET AL: "Differential effects of prone position in COVID-19-related ARDS in low and high recruiters", INTENSIVE CARE MEDICINE, SPRINGER BERLIN HEIDELBERG, BERLIN/HEIDELBERG, vol. 47, no. 9, 28 June 2021 (2021-06-28), pages 1044 - 1046, XP037556859, ISSN: 0342-4642, [retrieved on 20210628], DOI: 10.1007/S00134-021-06466-3**

EP 4 406 575 B1

(52) Classification Coopérative des Brevets (CPC):
(Cont.)A61M 2230/40; A61M 2230/46

**Description**

L'invention concerne un ventilateur médical, c'est-à-dire un appareil d'assistance respiratoire, permettant de fournir un diagnostic clinique en temps réel d'un patient ventilé atteint d'une détresse respiratoire, c'est-à-dire d'un SDRA, sous forme d'un index de recrutabilité des poumons du patient considéré, à savoir un rapport recrutement/inflation ou rapport de recrutabilité, appelé R-I ratio.

Le syndrome de détresse respiratoire aiguë ou SDRA est une cause fréquente de mortalité chez l'homme. Un SDRA se caractérise par une atteinte sévère des poumons dont seulement une faible partie peut être recrutée, c'est-à-dire être ventilée et apte à participer aux échanges gazeux pulmonaires, à savoir apport d'oxygène ($O_2$) inspiré et élimination du dioxyde de carbone ($CO_2$) expiré. Les patients atteints de SDRA ont ce que l'on appelle des « baby lungs » ou « poumons de bébés ».

La prise en charge ventilatoire du SDRA nécessite l'application d'une ventilation dite « protectrice », par exemple au moyen d'un ventilateur médical comme celui décrit par US 2022/0096765, ainsi qu'une personnalisation des paramètres ventilatoires pour les adapter au patient considéré.

Ainsi, une augmentation de la pression expiratoire positive ou PEP (aussi appelée PEEP en anglais pour Positive End-Expiratory Pressure) par rapport à un niveau de PEP préalablement réglé peut permettre de recruter une partie supplémentaire des poumons, c'est-à-dire d'ouvrir des parties du poumon qui ne participaient pas aux échanges gazeux avant l'application de cette PEP. Cependant, une PEP trop élevée pourrait également distendre une partie des poumons et être délétère pour le patient.

Selon une définition courante, la PEP peut être définie comme la pression gazeuse résiduelle maintenue au-dessus de la pression atmosphérique, dans les voies aériennes d'une personne, i.e. d'un patient, en fin d'expiration. La PEP permet de ventiler les alvéoles pulmonaires qui pourraient être collabées et d'une manière générale, permet d'augmenter les échanges gazeux pulmonaires et de diminuer le travail respiratoire du patient.

La capacité à recruter les poumons en augmentant la PEP dépend des individus, c'est-à-dire des patients à traiter. On distingue ainsi :

- les patients dits « recruteurs » pour lesquels une augmentation de PEP permet de recruter une partie supplémentaire des poumons. Ce recrutement, lorsqu'il est efficace, permet d'améliorer les échanges gazeux et s'avère bénéfique pour les patients.
- les patients dits « non recruteurs » pour lesquels une augmentation de PEP ne permet pas de recruter une partie supplémentaire des poumons et peut même être délétère pour le patient puisque susceptible de conduire notamment à une distension des poumons, une instabilité hémodynamique...

On comprend donc qu'il est primordial de pouvoir évaluer la recrutabilité de chaque patient afin de personnaliser sa ventilation, notamment le réglage de la PEP à lui appliquer, pour assurer une ventilation efficace du patient et éviter une ventilation délétère si le patient est non recruteur.

Si différentes méthodes ont été proposées pour déterminer le réglage de la PEP, par exemple la méthode des courbes pression/volume multiples, l'utilisation de l'électro-impédance par tomographie (EIT), l'adaptation de la PEP en fonction de la valeur de la fraction inspirée en oxygène ($FiO_2$) réglée sur le ventilateur ou en fonction de la mécanique respiratoire du patient (i.e. pression motrice, pression de plateau...)..., celles-ci donnent en général des résultats aléatoires et souvent difficiles à mettre en œuvre en pratique, par exemple en milieu hospitalier au lit du malade.

Par ailleurs, on connait aussi le document Chen Lu et al, Potential for Lung Recruitment Estimated by the Recruitment-To-Infliation Ratio in Acute Respiratory Distress Syndrom ; A Clinical Trial; American Journal of Respiratory and Care Medecine; vol. 201, n°2, 15 janv. 2020; p. 178-187, qui enseigne des essais visant à évaluer le recrutement pulmonaire chez des patients souffrant de SDRA. Les patients sont ventilés par un ventilateur médical mais les mesures sont traitées par un ordinateur indépendant qui est connecté au ventilateur, et qui met en œuvre un logiciel particulier, à savoir MATLAB (cf. parag. Analysis, p.182).

Cette manière de procéder est peu pratique car elle requiert d'utiliser un ordinateur et des connexions entre ordinateur et ventilateur.

Un problème est donc de pouvoir fournir une évaluation fiable de la recrutabilité d'un patient atteint de SDRA ventilé par un ventilateur médical afin de pouvoir personnaliser sa ventilation, notamment le réglage de la PEP à lui appliquer, pour lui assurer une ventilation efficace non-délétère, en particulier si le patient est non-recruteur, et ce, sans rencontrer les inconvénients susmentionnés.

Une solution concerne un ventilateur médical, c'est-à-dire un appareil d'assistance respiratoire, comprenant :

- un circuit de gaz comprenant une branche inspiratoire pour acheminer un gaz respiratoire et une branche expiratoire pour évacuer le gaz expiré vers l'atmosphère,
- des moyens de mesure du débit configurés pour déterminer au moins un débit gazeux dans le circuit de gaz,

- des moyens de mesure de la pression configurés pour déterminer au moins une pression gazeuse dans le circuit de gaz,
- une valve de PEP agencée sur la branche expiratoire,
- un afficheur graphique, et
- des moyens de pilotage à microprocesseur coopérant avec les moyens de mesure du débit, les moyens de mesure de la pression, la valve de PEP et l'afficheur graphique.

De plus, selon l'invention :

- les moyens de pilotage du ventilateur médical sont configurés pour :

a) déterminer un R-I ratio à partir des mesures de débit et de pression opérées par les moyens de mesure du débit et les moyens de mesure de la pression, où le R-I ratio est tel que :

$$\text{R-I ratio} = \text{Crec} / \text{Crs}$$

où :

- Crec est la compliance des poumons recrutés du patient et
- Crs est la compliance du système respiratoire,

b) commander un affichage sur l'afficheur graphique, du R-I ratio calculé,

- et l'afficheur graphique est configuré pour afficher le R-I ratio.

Selon l'invention, on appelle :

- PEP : la pression gazeuse résiduelle maintenue par un ventilateur médical au-dessus de la pression atmosphérique (i.e. 1 atm), dans le circuit patient du ventilateur, donc indirectement aussi dans les voies aériennes d'une personne, i.e. d'un patient, en fin d'expiration, c'est-à-dire en fin de chacune de ses phases expiratoires.
- « R-I ratio » : le rapport recrutement/inflation des poumons d'une personne, typiquement un patient (pour *recruitment to inflation ratio* en anglais) qui estime la capacité d'une partie supplémentaire des poumons à être recrutée, c'est-à-dire ouverte, pour participer aux échanges gazeux par mise en œuvre d'une PEP.
- patient : une personne ou être humain (e.g. homme, femme...) qu'il soit un adulte, un adolescent, un enfant, un bébé, un nouveau-né ou autre atteint d'un trouble ou d'une pathologie respiratoire, tel un SDRA ou autre, nécessitant une ventilation artificielle.

Selon l'invention, la détermination du R-I ratio est opéré directement dans le ventilateur médical et ne requiert aucun ordinateur ou autre appareil informatique relié au ventilateur, servant à cette détermination. Autrement dit, selon l'invention, la détermination du R-I ratio se fait automatiquement aux sein des moyens de pilotage du ventilateur médical.

Intégrer cette détermination du R-I ratio à un ventilateur médical n'avait jamais été fait ou suggéré jusqu'à présent. Elle constitue une avance importante dans le traitement des patients SDRA. En effet, il est désormais possible d'évaluer la recrutabilité de chaque patient grâce au ventilateur médical lui-même, c'est-à-dire en temps réel pendant une ventilation appliquée au patient, et donc de pouvoir rétroagir immédiatement au niveau dudit ventilateur afin de personnaliser la ventilation appliquée au patient considéré, en particulier le réglage de la PEP qui lui est appliquée. Ceci assure une ventilation efficace et non-délétère même lorsque le patient est non-recruteur.

Selon le mode de réalisation considéré, le ventilateur médical de l'invention peut comprendre l'une ou plusieurs des caractéristiques suivantes :

- les moyens de pilotage sont configurés pour déterminer la compliance (Crs) du système respiratoire (Crs) telle que :
Crs = $V$te / (P$_{plat}$ - PEP$_{basse\_mesurée}$)
où :

  ∘ P$_{plat}$ est la pression de plateau à PEP$_{basse}$, où : PEP$_{basse}$ = PEP$_{haute}$ - A avec PEP$_{haute}$ comprise entre 10 et 30 cmH2O et A compris entre 5 et 15 cmH2O,
  ∘ PEP$_{basse\_mesurée}$ est une valeur de PEP basse mesurée par le capteur de pression, et
  ∘ Vte est le volume courant expiré par le patient (à PEP$_{basse}$).

- les moyens de pilotage sont configurés pour déterminer la compliance (Crec) des poumons recrutés du patient telle que :

$$Crec = \Delta Vrec / (PEP_{haute\_mesurée} - PEP_{basse\_mesurée})$$

où $\Delta Vrec$ est le volume gazeux recruté avec :

$$\Delta Vrec = \Delta EELV_{mesuré} - \Delta EELV_{estimé} - Vte$$

où :

o $\Delta EELV_{mesuré}$ est le volume gazeux $\Delta EELV_{mesuré}$ mesuré et généré par la transition entre $PEP_{haute}$ et $PEP_{basse}$, et
o $\Delta EELV_{estimé} = Crs \cdot (PEP_{haute\_mesurée} - PEP_{basse\_mesurée})$.

- les moyens de pilotage sont en outre configurés pour déterminer un état de recrutabilité du patient en comparant le RI-ratio déterminé pour le patient considéré avec une valeur-seuil de recrutabilité donnée, e.g. préfixée, et en déterminant que :

i) le patient est non-recruteur lorsque le R-I ratio est inférieur ou égal à la valeur-seuil de recrutabilité, ou
ii) le patient est recruteur lorsque le R-I ratio est supérieur à la valeur-seuil de recrutabilité.

- la valeur-seuil de recrutabilité est ajustable, c'est-à-dire modifiable par l'utilisateur.
- la valeur-seuil de recrutabilité est comprise entre 0 et 2.
- la valeur-seuil de recrutabilité est égale à 0,5.
- les moyens de pilotage sont en outre configurés pour commander un affichage sur l'afficheur graphique, de l'état de recrutabilité du patient.
- l'afficheur graphique est configuré pour afficher l'état de recrutabilité du patient.
- les moyens de pilotage sont configurés pour déterminer le R-I ratio calculé, après activation par l'utilisateur d'un moyen de sélection affiché sur l'afficheur graphique, de préférence par appui digital sur une touche virtuelle ou un pavé numérique.
- les moyens de pilotage sont en outre configurés pour déterminer une recommandation d'action à mener en fonction de l'état de recrutabilité ayant été déterminé et commander en outre un affichage sur l'afficheur graphique, de la recommandation d'action à mener.
- l'afficheur graphique est configuré pour afficher la recommandation d'action à mener.
- il comprend des moyens de mémorisation configurés pour mémoriser des recommandations d'action à mener comprenant une augmentation de la PEP, une diminution de la PEP ou un maintien de la PEP.
- il comprend en outre une micro-soufflante en tant que source de gaz alimentant la branche inspiratoire du circuit de gaz en air ou mélange air/oxygène.
- les moyens de pilotage comprennent un (ou des) microprocesseur(s).
- les moyens de pilotage comprennent (au moins) une carte électronique portant le (ou les) microprocesseur(s).
- le (les) microprocesseur met en œuvre un ou des algorithmes.
- il comprend des moyens de mémorisation, telle une mémoire informatique.
- les moyens de mémorisation sont agencés sur la carte électronique.
- il comprend des moyens d'alimentation électrique.
- les moyens d'alimentation électrique comprennent un cordon électrique et une prise de raccordement au secteur (110/220 V) et/ou une (des) batterie rechargeable, et éventuellement un transformateur de courant.
- l'afficheur graphique est un écran numérique tactile, i.e. à dalle numérique tactile, c'est-à-dire pouvant être commandé par des appuis digitaux de la part de l'utilisateur sur la dalle de l'écran, pour y effectuer des choix, des sélections, des entrées, des validations, lancer des actions, activer des fonctionnalités... ou opérer d'autres actions.
- l'écran d'affichage est du type à affichage en couleurs.
- l'écran d'affichage est configuré pour afficher des moyens de sélection, tel un pavé numérique ou une ou des touches de sélection digitales.
- il comprend une source de gaz sous pression pour fournir un débit de gaz à la branche inspiratoire.
- la source de gaz sous pression est une micro-soufflante motorisée, c'est-à-dire à moteur électrique.
- les moyens de pilotage sont configurés pour piloter la micro-soufflante.

- les moyens de pilotage sont configurés pour piloter le fonctionnement de la micro-soufflante, en particulier les accélérations ou décélérations de la micro-soufflante.
- les moyens d'alimentation électrique alimentent en courant électrique les composants requérant du courant électrique pour fonctionner, comme les moyens de pilotage, la micro-soufflante motorisée, l'écran d'affichage ou d'autres éléments.
- les moyens de pilotage sont en outre configurés pour commander un affichage sur l'afficheur graphique d'au moins une information additionnelle choisie parmi le volume courant (Vte), la fréquence respiratoire (Fr) et la PEP.
- l'afficheur graphique est en outre configuré pour opérer un affichage de ladite au moins une information additionnelle.

L'invention va maintenant être mieux comprise grâce à la description détaillée suivante, faite à titre illustratif mais non limitatif, en référence aux figures annexées parmi lesquelles :

Fig. 1 schématise un mode de réalisation de l'architecture interne d'un ventilateur médical selon l'invention ; et
Fig. 2 schématise un exemple d'affichage sur l'écran d'affichage d'un ventilateur médical selon l'invention.

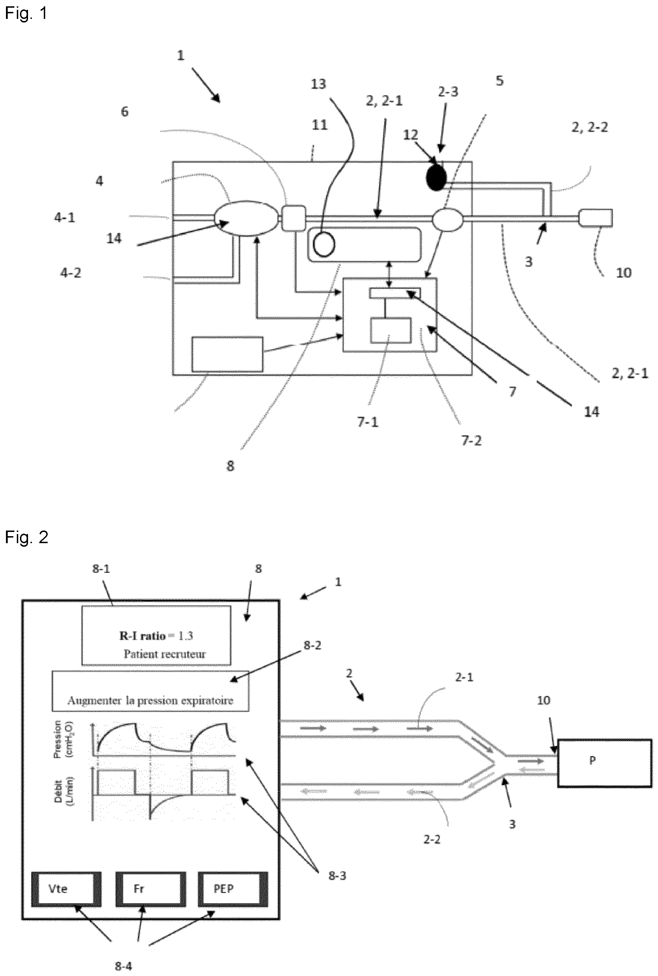

Fig. 1 schématise un mode de réalisation de l'architecture interne d'un ventilateur médical 1 selon l'invention, c'est-à-dire un appareil d'assistance ventilatoire, utilisable pour traiter une personne, c'est-à-dire un patient, souffrant de SDRA ou d'autres pathologies respiratoires.

Le ventilateur 1 fournit au patient un gaz respiratoire, tel qu'un mélange gazeux air/oxygène, pendant chaque phase inspiratoire du patient et rejette le gaz expiré provenant des poumons du patient pendant chaque phase expiratoire.

Le ventilateur est relié au patient via un circuit de gaz 2 qui est ici à double branche comprenant une branche inspiratoire 2-1 et une branche expiratoire 2-2, permettant l'acheminement du gaz fourni par une source de gaz.

La branche inspiratoire 2-1 et la branche expiratoire 2-2 viennent se raccorder fluidiquement à une pièce de jonction 3, telle une pièce en Y, permettant de faire la jonction entre les deux branches 2-1, 2-2. L'interface patient 10, tel un masque ou une sonde d'intubation, est reliée fluidiquement à la pièce de jonction 3.

L'apport d'oxygène ($O_2$) est assuré par la branche inspiratoire 2-1 et l'élimination du dioxyde de carbone ($CO_2$) expiré par le patient est assuré par la branche expiratoire 2-2 dont l'extrémité terminale abouti à l'atmosphère, via par exemple une sortie des gaz expirés 2-3 située au niveau de la carcasse 11 du ventilateur 1.

La branche expiratoire 2-2 comprend aussi une valve de PEP 12 agencée en amont de la sortie des gaz expirés 2-3 servant à régler le niveau de PEP pour assurer, selon l'invention, une ventilation efficace du patient et éviter une ventilation délétère si le patient est non-recruteur, comme expliqué ci-après

Le circuit de gaz 2 peut comprendre des passages de gaz internes agencés dans la carcasse ou coque externe 11 du ventilateur 1 et des conduits flexibles reliant le ventilateur 1 à l'interface respiratoire patient 10.

Le débit de gaz est fourni par une source de gaz sous pression 4, à savoir ici une micro-soufflante 14, aussi appelée turbine ou compresseur, aspirant l'air ambiant via une entrée d'air 4-1 du ventilateur 1 débouchant à l'atmosphère et par ailleurs alimentée en oxygène gazeux via une entrée d'oxygène 4-2 du ventilateur 1 par laquelle entre de l'oxygène provenant d'une source d'oxygène externe reliée au ventilateur 1 par un tuyau flexible, telle une bouteille d'oxygène sous pression, ou une prise murale de distribution d'oxygène alimentée en oxygène par une canalisation d'acheminement d'oxygène reliée à un stockage d'oxygène, ou encore un concentrateur d'oxygène.

Alternativement, la micro-soufflante 14 peut être remplacée par une valve de contrôle (non montrée) de l'alimentation en gaz agencée entre l'entrée d'air 4-1 du ventilateur 1 et la branche inspiratoire 2-1, par exemple en lieu et place de la micro-soufflante 14 sur Fig. 1. Dans ce cas, l'entrée d'air 4-1 du ventilateur 1 est alimentée par un conduit flexible connecté fluidiquement à une prise murale de distribution d'air médical porté par une paroi d'un bâtiment hospitalier et alimentée en air médical par une canalisation d'acheminement d'air médical reliée à un stockage d'air médical ou une installation de production d'air médical, telle une installation de type PSA, c'est-à-dire d'adsorption par pression modulée (Pressure Swing Adsorption) permettant de produire de l'air médical sur site, typiquement dans un bâtiment hospitalier, tel un hôpital, une clinique ou analogue.

Dans tous les cas, on obtient un mélange air/oxygène (teneur en $O_2$ >21% vol.) qui est acheminé jusqu'au patient via la branche inspiratoire 2-1 du circuit de gaz 2 du ventilateur 1.

Par ailleurs, sont prévus des moyens (i.e. un dispositif) de mesure de pression 5, tel un capteur de pression, et des moyens (i.e. un dispositif) de mesure de débit 6, tel un capteur de débit massique ou un capteur de pression différentiel, sont intégrés dans le ventilateur 1 sur le circuit de gaz 2 afin d'y déterminer une pression et un débit du gaz, i.e. du flux de mélange air/$O_2$ qui y circule.

Plus précisément, les moyens de mesure de pression 5 sont configurés et agencés pour déterminer au moins une pression du gaz, par exemple une pression reflétant la pression dans les voies aériennes du patient, alors que les moyens de mesure (i.e. de détermination) de débit 6 déterminent au moins une valeur de débit de gaz correspondant au débit du gaz dans le circuit de gaz 2 assurant l'acheminement du gaz provenant du ventilateur 1, c'est-à-dire typiquement dans la branche inspiratoire 2-1.

Des moyens ou une unité de pilotage 7 permettant d'opérer notamment les traitements de données, telles des mesures, et les différentes commandes du ventilateur 1 sont prévus dans le ventilateur 1. Ils comprennent au moins un microprocesseur 7-1, tel un microcontrôleur, mettant en œuvre un ou des algorithmes, de préférence portés par une carte électronique 7-2.

En outre, les moyens ou unité de pilotage 7 servent aussi à contrôler la micro-soufflante 14 (ou la valve de contrôle (non montrée) de l'alimentation en gaz assurant l'apport de gaz provenant d'une ou de plusieurs prises murales) afin de fournir le gaz pendant les phases inspiratoires du patient et diminuer ou stopper cet apport pendant ses phases expiratoires.

Par ailleurs, selon l'invention, les moyens ou unité de pilotage 7 pilotent aussi la valve de PEP 12 qui permet d'ajuster le niveau de PEP du patient, lors des expirations de gaz.

Le niveau de PEP souhaité est réglé par l'utilisateur sur l'IHM du ventilateur, c'est-à-dire le personnel soignant, comme expliqué ci-après. En général, la PEP est comprise entre 0 et 30 cmH2O, voire parfois au-delà, selon la catégorie de patient à traiter, à savoir adulte, adolescent, enfant, nouveau-né ou autre.

Les moyens de mesure de pression 5 et les moyens de mesure de débit 6 coopèrent avec les moyens de pilotage 7. Plus précisément, les moyens de mesure de pression 5 et de débit 6 fournissent les mesures de pression et de débit qu'ils opèrent, aux moyens de pilotage 7, lesquels réalisent alors un traitement de ces données, c'est-à-dire des mesures reçues, pour notamment déterminer les volumes générés par le ventilateur 1, c'est-à-dire délivrés par la source de gaz 4, par exemple le volume inspiré et le volume expiré, à partir des mesures successives de débit de gaz.

Les mesures de pression et de débit peuvent être des valeurs mesurées ou des signaux correspondant à des valeurs mesurées par les capteurs.

Le ventilateur 1 comprend aussi un afficheur graphique ou écran d'affichage 8 pour afficher des données, des informations, des courbes ou d'autres représentations graphiques, des alarmes, des réglages, des sélections, des menus, des icônes ou autres. L'affichage sur l'écran d'affichage 8 est commandé par les moyens de pilotage 7.

Par ailleurs, une source de courant électrique 9, i.e. des moyens d'alimentation électrique, est également prévue pour alimenter en courant électrique les différents composants du ventilateur 1 ayant besoin de courant électrique pour fonctionner, tels les moyens de pilotage 7, l'écran d'affichage 8, la micro-soufflante 14, les capteurs....

La source de courant électrique 9 peut comprendre une (des) batterie interne rechargeable qui peut être associée à un transformateur de courant, et/ou des moyens de raccordement au secteur (110/200 V), tel qu'un cordon électrique muni d'une prise de raccordement ou analogue.

L'écran d'affichage 8 est préférentiellement un écran numérique tactile, i.e. à dalle numérique tactile, c'est-à-dire pouvant être commandé par des appuis digitaux de la part de l'utilisateur sur la dalle de l'écran 8, pour y effectuer des choix, des sélections, des entrées, des validations, lancer des actions, activer des fonctionnalités... ou opérer d'autres actions. Avantageusement, l'écran d'affichage 8 est du type à affichage en couleurs.

De préférence, l'écran d'affichage 8 est configuré pour afficher des moyens de sélection, tel un pavé de monitorage spécifique, du mode de monitorage et/ou du rapport recrutement/inflation ou « R-I ratio » (pour *recruitment to inflation ratio* en anglais) selon l'invention, comme détaillé ci-après.

Un appui digital, i.e. tactile, de l'utilisateur, i.e. personnel soignant, tel un médecin, sur ce pavé de monitorage affiché sur la dalle de l'écran 8 permet de sélectionner ce mode et dès lors d'activer les différentes étapes du calcul automatique du R-I ratio, lesquelles sont données ci-après.

Autrement dit, l'appui de l'utilisateur sur le pavé de monitorage spécifique du mode R-I ratio affiché sur la dalle de l'écran 8 de l'écran d'affichage 8 est reconnu et transmis aux moyens de pilotage 7 qui opèrent alors un traitement de données adéquat, en particulier déterminent les actions à mener qui incluent le calcul du R-I ratio et son affichage subséquent sur l'écran d'affichage 8.

Le calcul du R-I ratio est fait alors automatiquement par les moyens de pilotage 7, comme détaillé ci-après.

D'une façon générale, les moyens de pilotage 7 à microprocesseur 7-1 coopèrent avec les moyens de mesure du débit 6, les moyens de mesure de la pression 5, la valve de PEP 12, l'afficheur graphique 8 et la source de gaz sous pression 4, telle la micro-soufflante 14 ou la valve de contrôle de l'alimentation en gaz (non montrée) servant à contrôler l'apport d'air provenant d'une prise murale par exemple.

Au sein des moyens de pilotage 7, le microprocesseur 7-1 est configuré pour calculer le R-I ratio de la manière suivante en commandant les différents composants du ventilateur 1 nécessaires à ce calcul.

Plus précisément, on opère comme suit :

a) modification ou fixation, typiquement par un médecin, de la fréquence respiratoire (Fr) de sorte que les moyens de pilotage 7, en particulier le microprocesseur 7-1, pilotent la micro-soufflante pour délivrer le gaz à une fréquence comprise entre 4 et 10 insufflations par minute; toutefois, selon un autre mode de réalisation, la fréquence peut être modifiée automatiquement par le ventilateur, et éventuellement validée par un médecin par appui sur une touche de validation par exemple,

b) passage à ou fixation, par contrôle de la valve expiratoire 12 par les moyens de pilotage 7, d'une PEP dite « haute »

ou PEP$_{haute}$ comprise entre 10 et 30 cmH2O, préférentiellement entre 10 et 25 cmH2O, par exemple de 15 cmH2O,

c) mesure par le capteur de pression 5 d'une PEP $_{haute\_mesurée}$,

d) diminution de la PEP de la valve expiratoire 12 par les moyens de pilotage 7, jusqu'à une PEP dite « basse » ou PEP$_{basse}$, telle que :

$$PEP_{basse} = PEP_{haute} - A$$

Où :

- PEP$_{haute}$ est comprise entre 10 et 30 cmH2O, par exemple 15 cmH2O,
- A est une valeur comprise entre 5 et 15 cmH2O, typiquement égale à environ 10 cmH2O

De là, on a par exemple PEP$_{basse}$ = 5 cmH2O (pour A = 10 cmH2O et PEP$_{haute}$=15 cmH2O),

e) détermination du volume gazeux $\Delta EELV_{mesuré}$ (End Expiratory Lung Volume ou volume pulmonaire de fin d'expiration) qui est mesuré et généré par la transition entre PEP$_{haute}$ et PEP$_{basse}$, le volume gazeux étant déterminé par les moyens de pilotage 7 par intégration du débit mesuré par les moyens de mesure de débit 6 pendant ladite transition.

f) mesure du volume gazeux courant (Vte) à PEP$_{basse}$, qui est le volume courant expiré par le patient (Vte = tidal volume expired), déterminé par les moyens de pilotage 7 par intégration du débit expiré mesuré par les moyens de mesure de débit 6 pendant le temps d'expiration à PEP$_{basse}$.

g) fixation d'un temps de pause inspiratoire ($t_{pi}$) pouvant aller jusqu'à 0.6 sec, typiquement compris entre 0.1 et 0.4 sec environ,

h) mesure par le capteur de pression 5 de la pression de plateau P$_{plat}$ obtenue à PEP$_{basse}$, c'est-à-dire la pression en fin d'inspiration à l'instant où le débit mesuré par les moyens de mesure de débit 6 est nul,

i) mesure par le capteur de pression 5 d'une PEP $_{basse\_mesurée}$,

j) calcul de la compliance Crs du système respiratoire à PEP$_{basse}$, où :

$$Crs = Vte / (P_{plat} - PEP_{basse\_mesurée})$$

k) estimation du volume $\Delta EELV_{estimé}$ estimé par une réduction de la PEP de PEP$_{haute}$ à PEP$_{basse}$ (e.g. entre 15 et 5 cmH2O) prédit par la compliance du système à PEP$_{basse}$, à savoir $\Delta_{estimé}$ = Crs * (PEP $_{haute\_mesurée}$ - PEP $_{basse\_mesurée}$),

l) calcul du volume gazeux recruté Vrec où :

$$\Delta Vrec = \Delta EELV_{mesuré} - \Delta EELV_{estimé} - Vte$$

m) calcul de la compliance des poumons recrutés Crec telle que :

$$Crec = \Delta Vrec / (PEP_{haute\_mesurée} - PEP_{basse\_mesurée})$$

n) calcul du rapport recrutement/inflation ou R-I ratio, tel que :

$$R\text{-}I\ ratio = Crec / Crs$$

Une fois le R-I ratio calculé par les moyens de pilotage 7, ces derniers commandent son affichage sur l'écran d'affichage 8, ainsi que préférentiellement une information sur l'état de recrutabilité du patient, par exemple sur un ou plusieurs pavés de monitorage 8-1,8-2 s'affichant sur l'écran d'affichage 8 du ventilateur 1.

Ainsi, Fig. 2 schématise un affichage sur l'écran d'affichage 8, d'un premier pavé de monitorage 8-1, c'est-à-dire une fenêtre d'affichage, permettant de visualiser le R-I ratio calculé par les moyens de pilotage 7 et l'état de recrutabilité du patient P.

L'état de recrutabilité du patient P est déterminé par les moyens de pilotage 7 de la manière suivante à partir du RI-ratio :

- si le R-I ratio est inférieur ou égal à une valeur-seuil, par exemple 0.5, les moyens de pilotage 7 déterminent que le patient est non-recruteur,
- à l'inverse, si le R-I ratio est supérieur à la valeur-seuil, les moyens de pilotage 7 déterminent que le patient est recruteur.

La valeur-seuil est fixée par défaut sur le ventilateur 1, peut être ajustée par l'utilisateur, par exemple un médecin au niveau de l'IHM, et mémorisée dans une mémoire 14 du ventilateur 1 et/ou dans un microprocesseur 7-1 des moyens de pilotage 7. Avantageusement, la valeur-seuil est comprise entre 0 et 2, notamment en fonction de la décision du médecin, typiquement la valeur-seuil est égale à 0.5, mais bien sûr, selon le cas, elle pourrait être supérieure à 0.5, par exemple égale à 0.6, 0.7, 0.8, 0.9 ou 1, voire plus.

Comme déjà dit, les moyens de pilotage 7 peuvent alors commander un affichage, i.e. visualisation, sur l'écran d'affichage 8, d'une information relative à l'état de recrutabilité du patient ayant été ainsi déterminé, typiquement un texte signalant cet état de recrutabilité du patient, par exemple "patient recruteur" ou "patient non-recruteur", comme illustré en Fig. 2, et/ou une représentation graphique avec un code de couleur dédié à chaque état, comme un visage de patient de couleur verte pour un patient recruteur et de couleur rouge pour un patient non-recruteur, ou toute information équivalente permettant au personnel soignant de distinguer immédiatement et/ou facilement un patient recruteur d'un patient non-recruteur.

Avantageusement, les moyens de pilotage 7 peuvent aussi être configurés pour déterminer et commander un affichage sur l'écran d'affichage 8, d'une recommandation d'action à mener par le personnel soignant en fonction de l'état de recrutabilité ayant été déterminé pour un patient donné, par exemple dans un deuxième pavé de monitorage 8-1, comme schématisé en Fig. 2, en particulier :

- pour un patient non-recruteur, proposer ou préconiser un maintien ou une diminution de la valeur de PEP fixée, ou
- pour un patient recruteur, proposer ou recommander une augmentation de la valeur de PEP et/ou une valeur-seuil de PEP maximale à ne pas dépasser.

Comme illustré en Fig. 2, la recommandation d'action à mener par le personnel soignant qui s'affiche sur l'écran d'affichage 8 peut être par exemple une information textuelle du type "maintien de PEP", "augmentation de PEP" ou "diminution de PEP", ou toute indication analogue ou équivalente.

Elle peut s'accompagner d'un affichage d'une représentation graphique permettant au personnel soignant de mieux visualiser l'action à mener recommandée, comme une flèche orientée vers le haut pour indiquer une augmentation de la pression positive ou à l'inverse, vers le bas pour indiquer une diminution de la pression positive, ou encore un signe « = » pour un maintien ou une conservation de la pression positive actuelle.

De plus, les moyens de pilotage 7 peuvent aussi être configurés pour commander un affichage sur l'écran d'affichage 8 :

- d'une représentation graphique et/ou la valeur du R-I ratio ayant été déterminée sous forme d'une valeur sans unité comprise entre 0 et 5, et/ou
- d'une (ou des) courbe(s) en temps réel (8-3) de type pression ou volume obtenue à partir de plusieurs mesures successives de pression de gaz et/ou de débit de gaz traitées par le microprocesseur des moyens de pilotage 7, comme illustré en Fig. 2.

En outre, d'autres informations utiles au personnel soignant peuvent être affichées (8-4) sur l'écran d'affichage 8, comme le volume courant (Vte), la fréquence respiratoire (Fr), la pression expiratoire ou PEP, ou d'autres paramètres ventilatoires, comme visible en Fig. 2.

Un ventilateur 1 selon l'invention est d'une grande aide pour le personnel soignant car il lui fournit une évaluation fiable de la recrutabilité d'un patient atteint de SDRA, grâce au calcul et à l'affichage du rapport recrutement/inflation ou R-I ratio, lui permettant alors de personnaliser la ventilation du patient considéré, en particulier le réglage de la PEP à lui appliquer, pour assurer une ventilation efficace non-délétère, que le patient soit recruteur ou non-recruteur.

D'une façon générale, un ventilateur médical 1 de l'invention peut être utilisé pour ventiler une personne, i.e. un patient, atteint d'une détresse respiratoire, i.e. un SDRA, en fournissant au personnel soignant une (des) indication de recrutabilité ou non des poumons du patient considéré et préférentiellement aussi une recommandation d'action à mener quant à la PEP à mettre en œuvre.

Des telles informations permettent d'aider le personnel soignant, i.e. médecin ou analogue, à adapter le réglage de la PEP, de préférence en association avec d'autres données ou paramètres cliniques disponibles, tels que courbes en temps réel de pression, de volume et/ou de débit ou autres.

De plus, un tel ventilateur présente l'avantage de permettre de donner une indication de la possibilité des poumons d'un patient à s'ouvrir davantage pour participer aux échanges gazeux lors d'une mise en œuvre d'une PEP à l'expiration et ce, directement au lit du patient et sans nécessité de matériel supplémentaire.

**Revendications**

1.  Ventilateur médical (1) comprenant :

    - un circuit de gaz (2) comprenant une branche inspiratoire (2-1) pour acheminer un gaz respiratoire et une branche expiratoire (2-2) pour évacuer le gaz expiré vers l'atmosphère,
    - des moyens de mesure du débit (6) configurés pour déterminer au moins un débit gazeux dans le circuit de gaz (2),
    - des moyens de mesure de la pression (5) configurés pour déterminer au moins une pression gazeuse dans le circuit de gaz (2),
    - une valve de PEP (12) agencée sur la branche expiratoire (2-2),
    - un afficheur graphique (8), et
    - des moyens de pilotage (7) à microprocesseur (7-1) coopérant avec les moyens de mesure du débit (6), les moyens de mesure de la pression (5), la valve de PEP (12) et l'afficheur graphique (8),

    **caractérisé en ce que** :

    - les moyens de pilotage (7) sont configurés pour :

        a) déterminer un R-I ratio à partir des mesures de débit et de pression opérées par les moyens de mesure du débit (6) et les moyens de mesure de la pression (5), où le R-I ratio est tel que :

        $$R\text{-}I\ ratio = Crec\ /\ Crs$$

        où :

        - Crec est la compliance des poumons recrutés du patient et
        - Crs est la compliance du système respiratoire,

        b) commander un affichage sur l'afficheur graphique (8), du R-I ratio calculé,

    - et l'afficheur graphique (8) est configuré pour afficher (8-1) le R-I ratio.

2.  Ventilateur selon la revendication 1, **caractérisé en ce que** les moyens de pilotage (7) sont configurés pour déterminer la compliance (Crs) du système respiratoire (Crs) telle que : $Crs = Vte\ /\ (P_{plat} - PEP_{basse\_mesurée})$, où :

    - $P_{plat}$ est la pression de plateau à $PEP_{basse}$, avec : $PEP_{basse} = PEP_{haute} - A$ et $PEP_{haute}$ comprise entre 10 et 30 cmH2O et A compris entre 5 et 15 cmH2O,
    - $PEP_{basse\_mesurée}$ est une valeur de PEP basse mesurée par le capteur de pression (5), et
    - Vte est le volume courant expiré par le patient.

3.  Ventilateur selon la revendication 2, **caractérisé en ce que** les moyens de pilotage (7) sont configurés pour déterminer la compliance (Crec) des poumons recrutés du patient telle que :

    $$Crec = \Delta Vrec\ /\ PEP_{haute\_mesurée} - PEP_{basse\_mesurée})$$

    où : $\Delta Vrec$ est le volume gazeux recruté avec :

    $$\Delta Vrec = \Delta EELV_{mesuré} - \Delta EELV_{estimé} - Vte$$

    où :

    - $\Delta EELV_{mesuré}$ est le volume gazeux $\Delta EELV_{mesuré}$ mesuré et généré par la transition entre $PEP_{haute}$ et $PEP_{basse}$, et
    - $\Delta EELV_{estimé} = Crs\ .\ (PEP_{haute\_mesurée} - PEP_{basse\_mesurée})$.

**4.** Ventilateur selon l'une des revendications précédentes, **caractérisé en ce que** les moyens de pilotage (7) sont en outre configurés pour déterminer un état de recrutabilité du patient en comparant le RI-ratio déterminé pour le patient considéré avec une valeur-seuil de recrutabilité donnée et en déterminant que :

    - le patient est non-recruteur lorsque le R-I ratio est inférieur ou égal à la valeur-seuil de recrutabilité, ou
    - le patient est recruteur lorsque le R-I ratio est supérieur à la valeur-seuil de recrutabilité.

**5.** Ventilateur selon la revendication 4, **caractérisé en ce que** la valeur-seuil de recrutabilité est comprise entre 0 et 2, de préférence égale à 0,5.

**6.** Ventilateur selon la revendication 1, **caractérisé en ce que** :

    - les moyens de pilotage (7) sont en outre configurés pour commander un affichage sur l'afficheur graphique (8), de l'état de recrutabilité du patient (8-1) et
    - l'afficheur graphique (8) est configuré pour afficher (8-1) l'état de recrutabilité du patient.

**7.** Ventilateur selon la revendication 1, **caractérisé en ce que** les moyens de pilotage (7) sont configurés pour déterminer le R-I ratio calculé, après activation par l'utilisateur d'un moyen de sélection (13) affiché sur l'afficheur graphique (8).

**8.** Ventilateur selon la revendication 7, **caractérisé en ce que** l'activation du moyen de sélection (13) est opéré par appui digital sur une touche virtuelle ou un pavé numérique.

**9.** Ventilateur selon la revendication 1, **caractérisé en ce que** :

    - les moyens de pilotage (7) sont en outre configurés pour déterminer une recommandation d'action à mener en fonction de l'état de recrutabilité ayant été déterminé et commander en outre un affichage (8-2) sur l'afficheur graphique (8), de la recommandation d'action à mener, et
    - l'afficheur graphique (8) est configuré pour afficher la recommandation d'action à mener (8-2).

**10.** Ventilateur selon la revendication 9, **caractérisé en ce qu'**il comprend des moyens de mémorisation (14) configurés pour mémoriser des recommandations d'action à mener comprenant une augmentation de la PEP, une diminution de la PEP ou un maintien de la PEP.

**11.** Ventilateur selon la revendication 1, **caractérisé en ce qu'**il comprend en outre une micro-soufflante (14) en tant que source de gaz (4) alimentant la branche inspiratoire (2-1) du circuit de gaz (2) en air ou mélange air/oxygène.

**12.** Ventilateur selon la revendication 1, **caractérisé en ce que** les moyens de pilotage comprennent au moins une carte électronique comprenant au moins un microprocesseur.

**13.** Ventilateur selon les revendications 11 et 12, **caractérisé en ce que** les moyens de pilotage sont configurés pour piloter la micro-soufflante (14).

**14.** Ventilateur selon la revendication 1, **caractérisé en ce que** les moyens de pilotage (7) sont en outre configurés pour commander un affichage (8-4) sur l'afficheur graphique (8) d'au moins une information additionnelle choisie parmi le volume courant (Vte), la fréquence respiratoire (Fr) et la PEP.

**15.** Ventilateur selon la revendication 1, **caractérisé en ce que** l'écran d'affichage (8) est du type à affichage en couleurs.

**Patentansprüche**

**1.** Medizinisches Beatmungsgerät (1), umfassend:

    - einen Gaskreis (2), umfassend einen Einatemzweig (2-1) zum Weiterleiten eines Atemgases und einen Ausatemzweig (2-2) zum Abführen des ausgeatmeten Gases zur Atmosphäre,
    - Volumenstrommessmittel (6), die dazu ausgestaltet sind, mindestens einen Gasvolumenstrom in dem Gaskreis (2) zu ermitteln,

- Druckmessmittel (5), die dazu ausgestaltet sind, mindestens einen Gasdruck in dem Gaskreis (2) zu ermitteln,
- ein PEP-Ventil (12), das an dem Ausatemzweig (2-2) angeordnet ist,
- eine grafische Anzeige (8), und
- Ansteuerungsmittel (7) mit Mikroprozessor (7-1), die mit den Volumenstrommessmitteln (6), den Druckmessmitteln (5), dem PEP-Ventil (12) und der grafischen Anzeige (8) zusammenwirken,

**dadurch gekennzeichnet, dass**:

- die Ansteuerungsmittel (7) dazu ausgestaltet sind:

a) ein R/I-Verhältnis ausgehend von den Volumenstrom- und Druckmessungen zu ermitteln, die von den Volumenstrommessmitteln (6) und den Druckmessmitteln (5) vorgenommen werden, wobei das R/I-Verhältnis dergestalt ist, dass:

$$\text{R/I-Verhältnis} = Crec/Crs$$

worin:

- Crec die Compliance der rekrutierten Lunge des Patienten ist und
- Crs die Compliance des respiratorischen Systems ist,

b) eine Anzeige des berechneten R/I-Verhältnisses auf der grafischen Anzeige (8) zu steuern,

- und die grafische Anzeige (8) dazu ausgestaltet ist, das R/I-Verhältnis anzuzeigen (8-1).

2. Beatmungsgerät nach Anspruch 1, **dadurch gekennzeichnet, dass** die Ansteuerungsmittel (7) dazu ausgestaltet sind, die Compliance (Crs) des respiratorischen Systems (Crs) dergestalt zu ermitteln, dass: $Crs = Vte / (P_{Plat} - PEP_{unterer\_gemessener}$, worin:

- $P_{Plat}$ der Plateaudruck bei $PEP_{unterer}$ ist, mit: $PEP_{unterer} = PEP_{oberer} - A$ und $PEP_{oberer}$ zwischen 10 und 30 cmH2O und A zwischen 5 und 15 cmH2O,
- $PEP_{unterer\_gemessener}$ ein von dem Drucksensor (5) gemessener unterer PEP-Wert ist, und
- Vte das von dem Patienten ausgeatmete aktuelle Volumen ist.

3. Beatmungsgerät nach Anspruch 2, **dadurch gekennzeichnet, dass** die Ansteuerungsmittel (7) dazu ausgestaltet sind, die Compliance (Crec) der rekrutierten Lunge des Patienten dergestalt zu ermitteln, dass:

$$Crec = \Delta Vrec / PEP_{oberer\_gemessener} - PEP_{unterer\_gemessener})$$

worin: $\Delta Vrec$ das rekrutierte Gasvolumen ist mit:

$$\Delta Vrec = \Delta EELV_{gemessen} - \Delta EELV_{geschätzt} - Vte$$

worin:

- $\Delta EELV_{gemessen}$ das gemessene und durch den Übergang zwischen $PEP_{oberer}$ und $PEP_{unterer}$ erzeugte Gasvolumen $\Delta EELV_{gemessen}$ ist, und
- $\Delta EELV_{geschätzt} = Crs \cdot (PEP_{oberer\_gemessener} - PEP_{unterer\_gemessener})$.

4. Beatmungsgerät nach einem der vorhergehenden Ansprüche, **dadurch gekennzeichnet, dass** die Ansteuerungsmittel (7) ferner dazu ausgestaltet sind, einen Rekrutierbarkeitszustand des Patienten zu ermitteln, indem das für den betrachteten Patienten ermittelte R/I-Verhältnis mit einem gegebenen Rekutierbarkeitsschwellenwert verglichen wird und indem ermittelt wird, dass:

- der Patient nicht-rekrutierend ist, wenn das R/I-Verhältnis kleiner als oder gleich dem Rekrutierbarkeitsschwellenwert ist, oder

- der Patient rekrutierend ist, wenn das R/I-Verhältnis größer als der Rekrutierbarkeitsschwellenwert ist.

5. Beatmungsgerät nach Anspruch 4, **dadurch gekennzeichnet, dass** der Rekrutierbarkeitsschwellenwert zwischen 0 und 2 liegt, bevorzugt 0,5 beträgt.

6. Beatmungsgerät nach Anspruch 1, **dadurch gekennzeichnet, dass**:

- die Ansteuerungsmittel (7) ferner dazu ausgestaltet sind, eine Anzeige des Rekrutierbarkeitszustands des Patienten (8-1) auf der grafischen Anzeige (8) zu steuern, und
- die grafische Anzeige (8) dazu ausgestaltet ist, den Rekrutierbarkeitszustand (8-1) des Patienten anzuzeigen.

7. Beatmungsgerät nach Anspruch 1, **dadurch gekennzeichnet, dass** die Ansteuerungsmittel (7) dazu ausgestaltet sind, das berechnete R/I-Verhältnis nach Aktivierung eines auf der grafischen Anzeige (8) angezeigten Auswahlmittels (13) durch den Benutzer zu ermitteln.

8. Beatmungsgerät nach Anspruch 7, **dadurch gekennzeichnet, dass** die Aktivierung des Auswahlmittels (13) durch Fingertippen auf eine virtuelle Taste oder einen Ziffernblock vorgenommen wird.

9. Beatmungsgerät nach Anspruch 1, **dadurch gekennzeichnet, dass**:

- die Ansteuerungsmittel (7) ferner dazu ausgestaltet sind, eine Handlungsempfehlung in Abhängigkeit von dem ermittelten Rekrutierbarkeitszustand zu ermitteln und ferner eine Anzeige (8-2) der Handlungsempfehlung auf der grafischen Anzeige (8) zu steuern, und
- die grafische Anzeige (8) dazu ausgestaltet ist, die Handlungsempfehlung (8-2) anzuzeigen.

10. Beatmungsgerät nach Anspruch 9, **dadurch gekennzeichnet, dass** es Speichermittel (14) umfasst, die dazu ausgestaltet sind, Handlungsempfehlungen zu speichern, die eine Erhöhung des PEP, eine Verringerung des PEP oder eine Beibehaltung des PEP umfassen.

11. Beatmungsgerät nach Anspruch 1, **dadurch gekennzeichnet, dass** es ferner ein Mikrogebläse (14) als Gasquelle (4) umfasst, die den Einatemzweig (2-1) des Gaskreises (2) mit Luft oder Luft/Sauerstoff-Gemisch versorgt.

12. Beatmungsgerät nach Anspruch 1, **dadurch gekennzeichnet, dass** die Ansteuerungsmittel mindestens eine Elektronikkarte umfassen, die mindestens einen Mikroprozessor umfasst.

13. Beatmungsgerät nach den Ansprüchen 11 und 12, **dadurch gekennzeichnet, dass** die Ansteuerungsmittel dazu ausgestaltet sind, das Mikrogebläse (14) anzusteuern.

14. Beatmungsgerät nach Anspruch 1, **dadurch gekennzeichnet, dass** die Ansteuerungsmittel (7) ferner dazu ausgestaltet sind, eine Anzeige (8-4) mindestens einer zusätzlichen Information, die aus dem aktuellen Volumen (Vte), der Atemfrequenz (Fr) und dem PEP gewählt ist, auf der grafischen Anzeige (8) zu steuern.

15. Beatmungsgerät nach Anspruch 1, **dadurch gekennzeichnet, dass** der Anzeigebildschirm (8) vom Typ mit Farbanzeige ist.

**Claims**

1. Medical ventilator (1) comprising:

- a gas circuit (2) comprising an inspiratory branch (2-1) for conveying a respiratory gas and an expiratory branch (2-2) for evacuating the exhaled gas to the atmosphere,
- flow rate measurement means (6) configured to determine at least one gaseous flow rate in the gas circuit (2),
- pressure measurement means (5) configured to determine at least one gaseous pressure in the gas circuit (2),
- a PEEP valve (12) arranged on the expiratory branch (2-2),
- a graphical display (8), and
- operating means (7) with microprocessor (7-1) cooperating with the flow rate measurement means (6), the pressure measurement means (5), the PEEP valve (12) and the graphical display (8),

**characterized in that**:

- the operating means (7) are configured to:

a) determine an R-I ratio from the flow rate and pressure measurements performed by the flow rate measurement means (6) and the pressure measurement means (5), where the R-I ratio is such that:

$$R\text{-}I \text{ ratio} = Crec \; / \; Crs$$

where:

- Crec is the compliance of the recruited lungs of the patient and
- Crs is the compliance of the respiratory system,

b) order a display, on the graphical display (8), of the calculated R-I ratio,

- and the graphical display (8) is configured to display (8-1) the R-I ratio.

2. Ventilator according to Claim 1, **characterized in that** the operating means (7) are configured to determine the compliance (Crs) of the respiratory system (Crs) such that: $Crs = Vte / (P_{plat} - PEEP \text{ measured }_{low})$, where:

- $P_{plat}$ is the plateau pressure at $PEEP_{low}$, with: $PEEP_{low} = PEEP_{high} - A$ and $PEEP_{high}$ between 10 and 30 cmH2O and A between 5 and 15 cmH2O,
- $PEEP_{\text{ measured low}}$ is a low PEEP value measured by the pressure sensor (5), and
- Vte is the tidal volume exhaled by the patient.

3. Ventilator according to Claim 2, **characterized in that** the operating means (7) are configured to determine the compliance (Crec) of the recruited lungs of the patient such that:

$$Crec = \Delta vrec \; / \; PEEP_{measured\ high} - PEEP_{measured\ low})$$

where: $\Delta Vrec$ is the gas volume recruited with:

$$\Delta Vrec = \Delta EELV_{measured} - \Delta EELV_{estimated} - Vte$$

where:

- $\Delta EELV_{measured}$ is the gas volume $\Delta EELV_{measured}$ measured and generated by the transition between $PEEP_{high}$ and $PEEP_{low}$, and
- $\Delta EELV_{estimated} = Crs. (PEEP_{\text{ measured high}} - PEEP_{\text{ measured low}})$.

4. Ventilator according to any one of the preceding claims, **characterized in that** the operating means (7) are further configured to determine a state of recruitability of the patient by comparing the RI ratio, determined for the patient in question, against a given recruitability threshold value and by determining that:

- the patient is a non-recruiter when the R-I ratio is less than or equal to the recruitability threshold value, or
- the patient is a recruiter when the R-I ratio is greater than the recruitability threshold value.

5. Ventilator according to Claim 4, **characterized in that** the recruitability threshold value is between 0 and 2, preferably equal to 0.5.

6. Ventilator according to Claim 1, **characterized in that**:

- the operating means (7) are further configured to order a display, on the graphical display (8), of the state of recruitability of the patient (8-1), and
- the graphical display (8) is configured to display (8-1) the state of recruitability of the patient.

7. Ventilator according to Claim 1, **characterized in that** the operating means (7) are configured to determine the calculated R-I ratio, after activation, by the user, of a selection means (13) displayed on the graphical display (8).

8. Ventilator according to Claim 7, **characterized in that** the activation of the selection means (13) is effected by pressing with a finger on a virtual key or a numeric keypad.

9. Ventilator according to Claim 1, **characterized in that**:

   - the operating means (7) are further configured to determine a recommendation for action to be taken as a function of the state of recruitability that has been determined and to further order a display (8-2), on the graphical display (8), of the recommendation for action to be taken, and
   - the graphical display (8) is configured to display the recommendation for action to be taken (8-2).

10. Ventilator according to Claim 9, **characterized in that** it comprises storage means (14) configured to store recommendations for action to be taken, including increasing the PEEP, decreasing the PEEP or maintaining the PEEP.

11. Ventilator according to Claim 1, **characterized in that** it further comprises a micro-blower (14) as a gas source (4) supplying the inspiratory branch (2-1) of the gas circuit (2) with air or an air/oxygen mixture.

12. Ventilator according to Claim 1, **characterized in that** the operating means comprise at least one electronic card comprising at least one microprocessor.

13. Ventilator according to Claims 11 and 12, **characterized in that** the operating means are configured to operate the micro-blower (14).

14. Ventilator according to Claim 1, **characterized in that** the operating means (7) are further configured to order a display (8-4), on the graphical display (8), of at least one additional item of information selected from the tidal volume (Vte), the respiratory rate (Fr) and the PEEP.

15. Ventilator according to Claim 1, **characterized in that** the display screen (8) is of the type with a colour display.

Fig. 1

Fig. 2

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20220096765 A

**Littérature non-brevet citée dans la description**

- **CHEN LU et al.** Potential for Lung Recruitment Estimated by the Recruitment-To-Infliation Ratio in Acute Respiratory Distress Syndrom ; A Clinical Trial;. *American Journal of Respiratory and Care Medecine;*, 15 January 2020, vol. 201 (2), 178-187